(19) Europäisches Patentamt
European Patent Office
Office européen des brevets

(11) EP 0 974 664 B1

(12) EUROPEAN PATENT SPECIFICATION

(45) Date of publication and mention of the grant of the patent:
**26.10.2005 Bulletin 2005/43**

(51) Int Cl.[7]: **C12N 15/57**, C12N 9/48, C07K 16/40, C12Q 1/68, G01N 33/50, G01N 33/53, A61K 38/48

(21) Application number: **98203614.7**

(22) Date of filing: **27.10.1998**

(54) **Human polypeptides and polynucleotides homolog to mouse testes-specific angiotensins converting enzyme**

Humane Polypeptide und Poylnucleotide mit einer Homologie zum Testis-spezifischen Angiotensin konvertierenden Enzyms

Polypeptides et polynucleotides humains ayant une homologie avec l'enzyme de conversion de l'angiotensine spécifique des testicules

(84) Designated Contracting States:
**BE CH DE DK FR GB IT LI NL**

(30) Priority: **13.05.1998 GB 9810373**
**18.08.1998 GB 9818009**

(43) Date of publication of application:
**26.01.2000 Bulletin 2000/04**

(73) Proprietor: **SMITHKLINE BEECHAM PLC**
**Brentford, Middlesex TW8 9GS (GB)**

(72) Inventors:
- **Southan, Christopher D., SmithKline Beecham Pharm. Harlow, Essex CM19 5AD (GB)**
- **Burgess, Nicola, SmithKline Beecham Pharm. Harlow, Essex CM19 5AD (GB)**

(74) Representative:
**Connell, Anthony Christopher et al**
**GlaxoSmithKline,**
**New Frontiers Science Park,**
**Third Avenue**
**Harlow, Essex CM19 5AW (GB)**

(56) References cited:
**WO-A-00/18899** **WO-A-97/48801**

- **DONOGHUE MARY ET AL: "A novel angiotensin-converting enzyme-related carboxypeptidase (ACE2) converts angiotensin I to angiotensin 1-9" CIRCULATION RESEARCH, GRUNE AND STRATTON, BALTIMORE, US, vol. 87, no. 5, 1 September 2000 (2000-09-01), pages e1-e9, XP002207553 ISSN: 0009-7330 -& DATABASE EMBL [Online] 16 August 2000 (2000-08-16) retrieved from EBI Database accession no. af291820 XP002224394**
- **TIPNIS ET AL: "A human homolog of Angiotensin-converting enzyme" JOURNAL OF BIOLOGICAL CHEMISTRY, AMERICAN SOCIETY OF BIOLOGICAL CHEMISTS, BALTIMORE, MD, US, vol. 275, no. 43, 27 October 2000 (2000-10-27), pages 33238-33243, XP002207551 ISSN: 0021-9258**
- **EHLERS M R W ET AL: "MOLECULAR CLONING OF HUMAN TESTICULAR ANGIOTENSIN-CONVERTING ENZYME: THE TESTIC ISOZYME IS IDENTICAL TO THE C-TERMINAL HALF OF ENDOTHELIAL ANGIOTENSIN-CONVERTING ENZYME" PROCEEDINGS OF THE NATIONAL ACADEMY OF SCIENCES OF USA, NATIONAL ACADEMY OF SCIENCE. WASHINGTON, US, vol. 86, no. 20, 1 October 1989 (1989-10-01), pages 7741-7745, XP000113802 ISSN: 0027-8424 -& DATABASE EMBL [Online] 24 April 1990 (1990-04-24) retrieved from EBI Database accession no. m26657 XP002224393**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention

This invention relates to newly identified polypeptides and polynucleotides encoding such polypeptides, to their use in identifying compounds which may be agonists, antagonists and /or inhibitors which are potentially useful in therapy, and to production of such polypeptides and polynucleotides.

### Background of the Invention

The drug discovery process is currently undergoing a fundamental revolution as it embraces 'functional genomics', that is, high throughput genome- or gene-based biology. This approach as a means to identify genes and gene products as therapeutic targets is rapidly superceding earlier approaches based on 'positional cloning'. A phenotype, that is a biological function or genetic disease, would be identified and this would then be tracked back to the responsible gene, based on its genetic map position.

Functional genomics relies heavily on high-throughput DNA sequencing technologies and the various tools of bioinformatics to identify gene sequences of potential interest from the many molecular biology databases now available. There is a continuing need to identify and characterise further genes and their related polypeptides/proteins, as targets for drug discovery.

### Summary of the Invention

The present invention relates to MPROT15, in particular MPROT15 polypeptides and MPROT15 polynucleotides, recombinant materials and methods for their production. In another aspect, the invention relates to methods for identifying agonists and antagonists/inhibitors, using the materials provided by the invention, which may be useful for the treatment of hypertension, cardiomyopathy, stroke, heart disease, neurodegeneration, Alzheimer's disease and diseases associated with peptide hormone or cytokine processing, hereinafter referred to as "the Diseases", amongst others.

### Description of the Invention

In a first aspect, the present invention relates to MPROT15 polypeptides. Such peptides include isolated polypetides comprising an amino acid sequence which has at least 70% identity, preferably at least 80% identity, more preferably at least 90% identity, yet more preferably at least 95% identity, most preferably at least 97-99% identity, to that of SEQ ID NO:2 over the entire length of SEQ ID NO:2. Such polypeptides include those comprising the amino acid of SEQ ID NO:2.

Further peptides of the present invention include isolated polypeptides in which the amino acid sequence has at least 70% identity, preferably at least 80% identity, more preferably at least 90% identity, yet more preferably at least 95% identity, most preferably at least 97-99% identity, to the amino acid sequence of SEQ ID NO:2 over the entire length of SEQ ID NO:2. Such polypeptides include the polypeptide of SEQ ID NO:2.

Further peptides of the present invention include isolated polypeptides encoded by a polynucleotide comprising the sequence contained in SEQ ID NO: 1.

Polypeptides of the present invention are believed to be members of the metalloprotease family of polypeptides. They are therefore of interest because metalloproteases play critical roles in some disease pathologies. Therfore designing or screening for selective protease antagonists or agonists can lead to the development of new drugs (Seife, C., Science 277, 1602-1603). These properties are hereinafter referred to as MPROT15 activity" or MPROT15 polypeptide activity" or "biological activity of MPROT15". Also included amongst these activities are antigenic and immunogenic activities of said MPROT15 polypeptides, in particular the antigenic and immunogenic activities of the polypeptide of SEQ ID NO:2. Preferably, a polypeptide of the present invention exhibits at least one biological activity of MPROT15.

The polypeptides of the present invention may be in the form of the "mature" protein or may be a part of a larger protein such as a precursor or a fusion protein. It is often advantageous to include an additional amino acid sequence which contains secretory or leader sequences, pro-sequences, sequences which aid in purification such as multiple histidine residues, or an additional sequence for stability during recombinant production.

The present invention also includes variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, 1-3, 1-2 or 1 amino acids are substituted, deleted, or added in any combination.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

In a further aspect, the present invention relates to MPROT15 polynucleotides. Such polynucleotides include isolated polynucleotides comprising a nucleotide sequence encoding a polypeptide which has at least 70% identity, preferably at least 80% identity, more preferably at least 90% identity, yet more preferably at least 95% identity, to the amino acid sequence of SEQ ID NO:2, over the entire length of SEQ ID NO:2. In this regard, polypeptides which have at least 97% identity are highly preferred, whilst those with at least 98-99% identity are more highly preferred, and those with at least 99% identity are most highly preferred. Such polynucleotides include a polynucleotide comprising the nucleotide sequence contained in SEQ ID NO:1 encoding the polypeptide of SEQ ID NO:2.

Further polynucleotides of the present invention include isolated polynucleotides comprising a nucleotide sequence which has at least 70% identity, preferably at least 80% identity, more preferably at least 90% identity, yet more preferably at least 95% identity, to SEQ ID NO:1 over the entire length of SEQ ID NO:1. In this regard, polynucleotides which have at least 97% identity are highly preferred, whilst those with at least 98-99% identiy are more highly preferred, and those with at least 99% identity are most highly preferred. Such polynucleotides include a polynucleotide comprising the polynucleotide of SEQ ID NO:1 as well as the polynucleotide of SEQ ID NO:1.

The invention also provides polynucleotides which are complementary to all the above described polynucleotides.

The nucleotide sequence of SEQ ID NO:1 shows homology with mouse testes-specific angiotensin converting enzyme (Howard et al. Mol.Cell.Biol., 10, 4294-302, 1990). The nucleotide sequence of SEQ ID NO: 1 is a cDNA sequence and comprises a polypeptide encoding sequence (nucleotide 1 to 2413) encoding a polypeptide of 805 amino acids, the polypeptide of SEQ ID NO:2. The nucleotide sequence encoding the polypeptide of SEQ ID NO:2 may be identical to the polypeptide encoding sequence contained in SEQ ID NO:1 or it may be a sequence other than the one contained in SEQ ID NO:1, which, as a result of the redundancy (degeneracy) of the genetic code, also encodes the polypeptide of SEQ ID NO:2. The polypeptide of the SEQ ID NO:2 is structurally related to other proteins of the metalloprotease family, having homology and/or structural similarity with human testes-specific angiotensin converting enzyme (Ehlers et al. Proc.Natl.Acad.Sci., 86(20) 7741-7745, 1989).

Preferred polypeptides and polynucleotides of the present invention are expected to have, *inter alia*, similar biological functions/properties to their homologous polypeptides and polynucleotides. Furthermore, preferred polypeptides and polynucleotides of the present invention have at least one MPROT15 activity.

The present invention also relates to partial or other polynucleotide and polypeptide sequences which were first identified prior to the determination of the corresponding full length sequences of SEQ ID NO:1 and SEQ ID NO:2.

Accordingly, in a further aspect, the present invention provides for an isolated polynucleotide which:

(a) comprises a nucleotide sequence which has at least 70% identity, preferably at least 80% identity, more preferably at least 90% identity, yet more preferably at least 95% identity, even more preferably at least 97-99% identity to SEQ ID NO:3 over the entire length of SEQ ID NO:3;
(b) has a nucleotide sequence which has at least 70% identity, preferably at least 80% identity, more preferably at least 90% identity, yet more preferably at least 95% identity, even more preferably at least 97-99% identity, to SEQ ID NO:3 over the entire length of SEQ ID NO:3;
(c) the polynucleotide of SEQ ID NO:3; or
(d) a nucleotide sequence encoding a polypeptide which has at least 70% identity, preferably at least 80% identity, more preferably at least 90% identity, yet more preferably at least 95% identity, even more preferably at least 97-99% identity, to the amino acid sequence of SEQ ID NO:4, over the entire length of SEQ ID NO:4; as well as the polynucleotide of SEQ ID NO:3.

The present invention further provides for a polypeptide which:

(a) comprises an amino acid sequence which has at least 70% identity, preferably at least 80% identity, more preferably at least 90% identity, yet more preferably at least 95% identity, most preferably at least 97-99% identity, to that of SEQ ID NO:4 over the entire length of SEQ ID NO:4;
(b) has an amino acid sequence which is at least 70% identity, preferably at least 80% identity, more preferably at least 90% identity, yet more preferably at least 95% identity, most preferably at least 97-99% identity, to the amino acid sequence of SEQ ID NO:4 over the entire length of SEQ ID NO:4;
(c) comprises the amino acid of SEQ ID NO:4; and
(d) is the polypeptide of SEQ ID NO:4; as well as polypeptides encoded by a polynucleotide comprising the sequence contained in SEQ ID NO:3.

The nucleotide sequence of SEQ ID NO:3 and the peptide sequence encoded thereby are derived from EST (Expressed Sequence Tag) sequences. It is recognised by those skilled in the art that there will inevitably be some nucleotide sequence reading errors in EST sequences (see Adams, M.D. *et al*, Nature 377 (supp) 3, 1995). Accordingly, the nucleotide sequence of SEQ ID NO:3 and the peptide sequence encoded therefrom are therefore subject to the same inherent limitations in sequence accuracy. Furthermore, the peptide sequence encoded by SEQ ID NO:3 comprises a region of identity or close homology and/or close structural similarity (for example a conservative amino acid difference) with the closest homologous or structurally similar protein.

Polynucleotides of the present invention may be obtained, using standard cloning and screening techniques, from a cDNA library derived from mRNA in cells of human skin, using the expressed sequence tag (EST) analysis (Adams, M.D., *et al*. Science (1991) 252:1651-1656; Adams, M.D. *et al*., Nature, (1992) *355*:632-634; Adams, M.D., *et al*., Nature (1995) 377 Supp:3-174). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself; or the coding sequence for the mature polypeptide in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further embodiments of the present invention include polynucleotides encoding polypeptide variants which comprise the amino acid sequence of SEQ ID NO:2 and in which several, for instance from 5 to 10, 1 to 5, 1 to 3, 1 to 2 or 1, amino acid residues are substituted, deleted or added, in any combination.

Polynucleotides which are identical or sufficiently identical to a nucleotide sequence contained in SEQ ID NO: 1, may be used as hybridization probes for cDNA and genomic DNA or as primers for a nucleic acid amplification (PCR) reaction, to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1. Typically these nucleotide sequences are 70% identical, preferably 80% identical, more preferably 90% identical, most preferably 95% identical to that of the referent. The probes or primers will generally comprise at least 15 nucleotides, preferably, at least 30 nucleotides and may have at least 50 nucleotides. Particularly preferred probes will have between 30 and 50 nucleotides. Particularly preferred primers will have between 20 and 25 nucleotides.

A polynucleotide encoding a polypeptide of the present invention may be obtained by a process which comprises the steps of screening an appropriate library under stringent hybridization conditions with a labeled probe having the sequence of SEQ ID NO:1 or a fragment thereof; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA; followed by washing the filters in 0.1x SSC at about 65°C.

The skilled artisan will appreciate that, in many cases, an isolated cDNA sequence will be incomplete, in that the region coding for the polypeptide is short at the 5' end of the cDNA. This is a consequence of reverse transcriptase, an enzyme with inherently low 'processivity' (a measure of the ability of the enzyme to remain attached to the template during the polymerisation reaction), failing to complete a DNA copy of the mRNA template during 1st strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., PNAS USA 85, 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end. Nucleic acid amplification (PCR) is then carried out to amplify the 'missing' 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, in a further aspect, the present invention relates to expression systems which comprise a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al, Basic Methods in Molecular Biology (1986) and Sambrook *et al*., Molecular Cloning: A Laboratory Manual, 2nd Ed., Cold Spring Harbor Laboratory Press, Cold Spring Harbor, N.Y. (1989). Preferred such methods include, for instance, calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *Streptococci*, *Staphylococci*, *E. coli*, *Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector which is able to maintain, propagate or express a polynucleotide to produce a polypeptide in a host may be used. The appropriate nucleotide sequence may be inserted into an expression system by any of a variety of well-known and routine techniques, such as, for example, those set forth in Sambrook *et al*., Molecular Cloning, A Laboratory Manual (supra). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and or purification.

The nucleotide sequences of the present invention are also valuable for chromosome localisation. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The gene of the present invention maps to human chromosome Xp22.

The nucleotide sequences of the present invention are also valuable for tissue localisation. Such techniques allow the determination of expression patterns of the human MPROT15 polypeptides in tissues by detection of the mRNAs that encode them. These techniques include in situ hybridziation techniques and nucleotide amplification techniques, for example PCR. Such techniques are well known in the art. Results from these studies provide an indication of the normal functions of the polypeptides in the organism. In addition, comparative studies of the normal expression pattern of human MPROT15 mRNAs with that of mRNAs encoded by a human MPROT15 gene provide valuable insights into the role of mutant human MPROT15 polypeptides, or that of inappropriate expression of normal human MPROT15 polypeptides, in disease. Such inappropriate expression may be of a temporal, spatial or simply quantitative nature.

The polypeptides of the invention or their fragments or analogs thereof, or cells expressing them, can also be used as immunogens to produce antibodies immunospecific for polypeptides of the present invention. The term "immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, analogs or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor *et al*., Immunology Today (1983) 4:72) and the EBV-hybridoma technique (Cole *et al*., Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography.

Antibodies against polypeptides of the present invention may also be employed to treat the Diseases, amongst others.

The polypeptides of the invention may also be used in a method for inducing an immunological response in a mammal which comprises inoculating the mammal with a polypeptide of the present invention, adequate to produce antibody and/or T cell immune response to protect said animal from the Diseases hereinbefore mentioned, amongst others. The polypeptides of the invention may also be used in a method of inducing immunological response in a mammal which comprises, delivering a polypeptide of the present invention *via* a vector directing expression of the polynucleotide and coding for the polypeptide *in vivo* in order to induce such an immunological response to produce antibody to protect said animal from diseases.

The polypeptides of the invention may also be used in an immunological/vaccine formulation (composition) which, when introduced into a mammalian host, induces an immunological response in that mammal to a polypeptide of the present invention wherein the composition comprises a polypeptide or polynucleotide of the present invention. The vaccine formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention are responsible for one or more biological functions, including one or more disease states, in particular the Diseases hereinbefore mentioned. It is therefore desirous to devise screening methods to identify compounds which stimulate or which inhibit the function of the polypeptide. Accordingly, in a further aspect, the present invention provides for a method of screening compounds to identify those which stimulate or which inhibit the function of the polypeptide. In general, agonists or antagonists may be employed for therapeutic and prophylactic purposes for such Diseases as hereinbefore mentioned. Compounds may be identified from a variety of sources, for example, cells, cell-free preparations, chemical libraries, and natural product mixtures. Such agonists, antagonists or inhibitors so-identified may be natural or modified substrates, ligands, receptors, enzymes, etc., as the case may be, of the polypeptide; or may be structural or functional mimetics thereof (see Coligan *et al*., Current Protocols in Immunology 1(2):Chapter 5 (1991)).

The screening method may simply measure the binding of a candidate compound to the polypeptide, or to cells or membranes bearing the polypeptide, or a fusion protein thereof by means of a label directly or indirectly associated with the candidate compound. Alternatively, the screening method may involve competition with a labeled competitor. Further, these screening methods may test whether the candidate compound results in a signal generated by activation or inhibition of the polypeptide, using detection systems appropriate to the cells bearing the polypeptide. Inhibitors of activation are generally assayed in the presence of a known agonist and the effect on activation by the agonist by the presence of the candidate compound is observed. Constitutively active polypeptides may be employed in screening methods for inverse agonists or inhibitors, in the absence of an agonist or inhibitor, by testing whether the candidate compound results in inhibition of activation of the polypeptide. Further, the screening methods may simply comprise the steps of mixing a candidate compound with a solution containing a polypeptide of the present invention, to form a mixture, measuring MPROT15 activity in the mixture, and comparing the MPROT15 activity of the mixture to a standard. Fusion proteins, such as those made from Fc portion and MPROT15 polypeptide, as hereinbefore described, can also be used for high-throughput screening assays to identify antagonists for the polypeptide of the present invention (see D. Bennett *et al*., J Mol Recognition, 8:52-58 (1995); and K. Johanson *et al*., J Biol Chem, 270(16): 9459-9471 (1995)).

The polynucleotides, polypeptides and antibodies to the polypeptide of the present invention may also be used to configure screening methods for detecting the effect of added compounds on the production ofmRNA and polypeptide in cells. For example, an ELISA assay may be constructed for measuring secreted or cell associated levels of polypeptide using monoclonal and polyclonal antibodies by standard methods known in the art. This can be used to discover agents which may inhibit or enhance the production of polypeptide (also called antagonist or agonist, respectively) from suitably manipulated cells or tissues.

The polypeptide may be used to identify membrane bound or soluble receptors, if any, through standard receptor binding techniques known in the art. These include, but are not limited to, ligand binding and crosslinking assays in which the polypeptide is labeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide which compete with the binding of the polypeptide to its receptors, if any. Standard methods for conducting such assays are well understood in the art.

Examples of potential polypeptide antagonists include antibodies or, in some cases, oligonucleotides or proteins which are closely related to the ligands, substrates, receptors, enzymes, etc., as the case may be, of the polypeptide, e.g., a fragment of the ligands, substrates, receptors, enzymes, etc.; or small molecules which bind to the polypeptide of the present invention but do not elicit a response, so that the activity of the polypeptide is prevented.

It will be readily appreciated by the skilled artisan that a polypeptide of the present invention may also be used in a method for the structure-based design of an agonist, antagonist or inhibitor of the polypeptide, by:

(a) determining in the first instance the three-dimensional structure of the polypeptide;
(b) deducing the three-dimensional structure for the likely reactive or binding site(s) of an agonist, antagonist or inhibitor;
(c) synthesing candidate compounds that are predicted to bind to or react with the deduced binding or reactive site; and
(d) testing whether the candidate compounds are indeed agonists, antagonists or inhibitors. It will be further appreciated that this will normally be an iterative process.

Agonists or antagonists identified using the screening method of the invention may be useful for treating abnormal conditions such as, for instance, hypertension, cardiomyopathy, stroke, heart disease, neurodegeneration, Alzheimer's disease and diseases associated with peptide hormone or cytokine processing,, related to either an excess of, or an under-expression of, MPROT15 polypeptide activity.

If the activity of the polypeptide is in excess, several approaches are available. One approach comprises administering to a subject in need thereof an inhibitor compound (antagonist) as hereinabove described, optionally in combination with a pharmaceutically acceptable carrier, in an amount effective to inhibit the function of the polypeptide, such as, for example, by blocking the binding of ligands, substrates, receptors, enzymes, etc., or by inhibiting a second signal, and thereby alleviating the abnormal condition. In another approach, soluble forms of the polypeptides still capable of binding the ligand, substrate, enzymes, receptors, etc. in competition with endogenous polypeptide may be administered. Typical examples of such competitors include fragments of the MPROT15 polypeptide.

For treating abnormal conditions related to an under-expression of MPROT15 and its activity, several approaches are also available. One approach comprises administering to a subject a therapeutically effective amount of a compound which activates a polypeptide of the present invention, i.e., an agonist as described above, in combination with a pharmaceutically acceptable carrier, to thereby alleviate the abnormal condition.

Agonists or antagonists may be used in pharmaceutical compositions comprising a therapeutically effective amount of a polypeptide, such as the soluble form of a polypeptide of the present invention, agonist/antagonist peptide or small molecule compound, in combination with a pharmaceutically acceptable carrier or excipient. Such carriers include, but are not limited to, saline, buffered saline, dextrose, water, glycerol, ethanol, and combinations thereof. Polypeptides and other compounds of the present invention may be employed alone or in conjunction with other compounds, such as therapeutic compounds.

The composition may be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of salves, pastes, gels, and the like.

The dosage range required depends on the choice of peptide or other compounds of the present invention, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, pgs. 1-12 in Post-translational Covalent Modification of Proteins, B. C. Johnson, Ed., Academic Press, New York, 1983; Seifter *et al*., "Analysis for protein modifications and nonprotein cofactors", Meth Enzymol (1990) 182: 626-646 and Rattan *et al*., "Protein Synthesis: Post-translational Modifications and Aging", Ann NY Acad Sci (1992) 663:48-62).

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in (*Computational Molecular Biology*, Lesk, A.M., ed., Oxford University Press, New York, 1988; *Biocomputing: Informatics and Genome Projects*, Smith, D.W., ed., Academic Press, New York, 1993; *Computer Analysis of Sequence Data*, Part 1, Griffin, A.M., and Griffin, H.G., eds., Humana Press, New Jersey, 1994; *Sequence Analysis in Molecular Biology*, von Heinje, G., Academic Press, 1987; and *Sequence Analysis Primer*, Gribskov, M. and Devereux, J., eds., M Stockton Press, New York, 1991; and Carillo, H., and Lipman, D., SIAM *J. Applied Math*., *48:* 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., *Nucleic Acids Research 12(1):* 387 (1984)), BLASTP, BLASTN, and FASTA (Atschul, S.F. et al., *J. Molec. Biol. 215:* 403-410 (1990). The BLAST X program is publicly available from NCBI and other sources (*BLAST Manual*, Altschul, S., *et al*., NCBI NLM NIH Bethesda, MD 20894; Altschul, S., *et al*., *J. Mol. Biol. 215:* 403-410 (1990). The well known Smith Waterman algorithm may also be used to determine identity.

Preferred parameters for polypeptide sequence comparison include the following:

1) Algorithm: Needleman and Wunsch, J. Mol Biol. 48: 443-453 (1970) Comparison matrix: BLOSSUM62 from Hentikoff and Hentikoff, Proc. Natl. Acad. Sci. USA. 89:10915-10919 (1992)

Gap Penalty: 12
Gap Length Penalty: 4

A program useful with these parameters is publicly available as the "gap" program from Genetics Computer Group, Madison WI. The aforementioned parameters are the default parameters for peptide comparisons (along with no penalty for end gaps).

Preferred parameters for polynucleotide comparison include the following:

1) Algorithm: Needleman and Wunsch, J. Mol Biol. 48: 443-453 (1970)

Comparison matrix: matches = +10, mismatch = 0
Gap Penalty: 50
Gap Length Penalty: 3
Available as: The "gap" program from Genetics Computer Group, Madison WI. These are the default parameters for nucleic acid comparisons.

By way of example, a polynucleotide sequence of the present invention may be identical to the reference sequence of SEQ ID NO:1, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO:1 by the numerical percent of the respective percent identity(divided by 100) and subtracting that product from said total number of nucleotides in SEQ ID NO:1, or:

$$n_n \leq x_n - (x_n \bullet y),$$

wherein $\mathbf{n_n}$ is the number of nucleotide alterations, $\mathbf{x_n}$ is the total number of nucleotides in SEQ ID NO:1, and **y** is, for instance, 0.70 for 70%, 0.80 for 80%, 0.85 for 85%, 0.90 for 90%, 0.95 for 95%,etc., and wherein any non-integer product of $\mathbf{x_n}$ and **y** is rounded down to the nearest integer prior to subtracting it from $\mathbf{x_n}$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

Similarly, a polypeptide sequence of the present invention may be identical to the reference sequence of SEQ ID NO:2, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the numerical percent of the respective percent identity(divided by 100) and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, and **y** is, for instance 0.70 for 70%, 0.80 for 80%, 0.85 for 85% etc., and wherein any non-integer product of $\mathbf{x_a}$ and **y** is rounded down to the nearest integer prior to subtracting it from $\mathbf{x_a}$.

"Homolog" is a generic term used in the art to indicate a polynucleotide or polypeptide sequence possessing a high degree of sequence relatedness to a subject sequence. Such relatedness may be quantified by determining the degree of identity and/or similarity between the sequences being compared as hereinbefore described. Falling within this generic term are the terms "ortholog", meaning a polynucleotide or polypeptide that is the functional equivalent of a polynucleotide or polypeptide in another species, and "paralog" meaning a functionally similar sequence when considered within the same species.

"Fusion protein" refers to a protein encoded by two, often unrelated, fused genes or fragments thereof In one example, EP-A-0 464 discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties [see, e.g., EP-A 0232 262]. On the other hand, for some uses it would be desirable to be able to delete the Fc part after the fusion protein has been expressed, detected and purified.

**SEQUENCE INFORMATION**

**SEQ ID NO:1**

ATGTCAAGCTCTTCCTGGCTCCTTCTCAGCCTTGTTGCTGTAACTGCTGCTCAGTCCACC
ATTGAGGAACAGGCCAAGACATTTTTGGACAAGTTTAACCACGAAGCCGAAGACCTGTTC
TATCAAAGTTCACTTGCTTCTTGGAATTATAACACCAATATTACTGAAGAGAATGTCCAA
AACATGAATAATGCTGGGGACAAATGGTCTGCCTTTTTAAAGGAACAGTCCACACTTGCC
CAAATGTATCCACTACAAGAAATTCAGAATCTCACAGTCAAGCTTCAGCTGCAGGCTCTT
CAGCAAAATGGGTCTTCAGTGCTCTCAGAAGACAAGAGCAAACGGTTGAACACAATTCTA
AATACAATGAGCACCATCTACAGTACTGGAAAAGTTTGTAACCCAGATAATCCACAAGAA
TGCTTATTACTTGAACCAGGTTTGAATGAAATAATGGCAAACAGTTTAGACTACAATGAG
AGGCTCTGGGCTTGGGAAAGCTGGAGATCTGAGGTCGGCAAGCAGCTGAGGCCATTATAT
GAAGAGTATGTGGTCTTGAAAAATGAGATGGCAAGAGCAAATCATTATGAGGACTATGGG
GATTATTGGAGAGGAGACTATGAAGTAAATGGGGTAGATGGCTATGACTACAGCCGCGGC
CAGTTGATTGAAGATGTGGAACATACCTTTGAAGAGATTAAACCATTATATGAACATCTT
CATGCCTATGTGAGGGCAAAGTTGATGAATGCCTATCCTTCCTATATCAGTCCAATTGGA
TGCCTCCCTGCTCATTTGCTTGGTGATATGTGGGGTAGATTTTGGACAAATCTGTACTCT
TTGACAGTTCCCTTTGGACAGAAACCAAACATAGATGTTACTGATGCAATGGTGGACCAG
GCCTGGGATGCACAGAGAATATTCAAGGAGGCCGAGAAGTTCTTTGTATCTGTTGGTCTT
CCTAATATGACTCAAGGATTCTGGGAAAATTCCATGCTAACGGACCCAGGAAATGTTCAG
AAAGCAGTCTGCCATCCCACAGCTTGGGACCTGGGGAAGGGCGACTTCAGGATCCTTATG
TGCACAAAGGTGACAATGGACGACTTCCTGACAGCTCATCATGAGATGGGGCATATCCAG
TATGATATGGCATATGCTGCACAACCTTTTCTGCTAAGAAATGGAGCTAATGAAGGATTC
CATGAAGCTGTTGGGGAAATCATGTCACTTTCTGCAGCCACACCTAAGCATTTAAAATCC
ATTGGTCTTCTGTCACCCGATTTTCAAGAAGACAATGAAACAGAAATAAACTTCCTGCTC
AAACAAGCACTCACGATTGTTGGGACTCTGCCATTTACTTACATGTTAGAGAAGTGGAGG
TGGATGGTCTTTAAAGGGGAAATTCCCAAAGACCAGTGGATGAAAAAGTGGTGGGAGATG
AAGCGAGAGATAGTTGGGGTGGTGGAACCTGTGCCCCATGATGAAACATACTGTGACCCC
GCATCTCTGTTCCATGTTTCTAATGATTACTCATTCATTCGATATTACACAAGGACCCTT
TACCAATTCCAGTTTCAAGAAGCACTTTGTCAAGCAGCTAAACATGAAGGCCCTCTGCAC
AAATGTGACATCTCAAACTCTACAGAAGCTGGACAGAAACTGTTCAATATGCTGAGGCTT
GGAAAATCAGAACCCTGGACCCTAGCATTGGAAAATGTTGTAGGAGCAAAGAACATGAAT
GTAAGGCCACTGCTCAACTACTTTGAGCCCTTATTTACCTGGCTGAAAGACCAGAACAAG
AATTCTTTTGTGGGATGGAGTACCGACTGGAGTCCATATGCAGACCAAAGCATCAAAGTG
AGGATAAGCCTAAAATCAGCTCTTGGAGATAAAGCATATGAATGGAACGACAATGAAATG
TACCTGTTCCGATCATCTGTTGCATATGCTATGAGGCAGTACTTTTTAAAAGTAAAAAAT
CAGATGATTCTTTTTGGGGAGGAGGATGTGCGAGTGGCTAATTTGAAACCAAGAATCTCC
TTTAATTTCTTTGTCACTGCACCTAAAAATGTGTCTGATATCATTCCTAGAACTGAAGTT
GAAAAGGCCATCAGGATGTCCCGGAGCCGTATCAATGATGCTTTCCGTCTGAATGACAAC
AGCCTAGAGTTTCTGGGGATACAGCCAACACTTGGACCTCCTAACCAGCCCCCTGTTTCC
ATATGGCTGATTGTTTTTGGAGTTGTGATGGGAGTGATAGTGGTTGGCATTGTCATCCTG
ATCTTCACTGGGATCAGAGATCGGAAGAAGAAAAATAAAGCAAGAAGTGGAGAAAATCCT
TATGCCTCCATCGATATTAGCAAAGGAGAAAATAATCCAGGATTCCAAAACACTGATGAT
GTTCAGACCTCCTTTTAG

## SEQ ID NO:2

MSSSSWLLLSLVAVTAAQSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLLLEPGLNEIMANSLDYNE
RLWAWESWRSEVGKQLRPLYEEYVVLKNEMARANHYEDYGDYWRGDYEVNGVDGYDYSRGQLIEDVEHTFEEIKPLYEHL
HAYVRAKLMNAYPSYISPIGCLPAHLLGDMWGRFWTNLYSLTVPFGQKPNIDVTDAMVDQAWDAQRIFKEAEKFFVSVGL
PNMTQGFWENSMLTDPGNVQKAVCHPTAWDLGKGDFRILMCTKVTMDDFLTAHHEMGHIQYDMAYAAQPFLLRNGANEGF
HEAVGEIMSLSAATPKHLKSIGLLSPDFQEDNETEINFLLKQALTIVGTLPFTYMLEKWRWMVFKGEIPKDQWMKKWWEM
KREIVGVVEPVPHDETYCDPASLFHVSNDYSFIRYYTRTLYQFQFQEALCQAAKHEGPLHKCDISNSTEAGQKLFNMLRL
GKSEPWTLALENVVGAKNMNVRPLLNYFEPLFTWLKDQNKNSFVGWSTDWSPYADQSIKVRISLKSALGDKAYEWNDNEM

YLFRSSVAYAMRQYFLKVKNQMILFGEEDVRVANLKPRISFNFFVTAPKNVSDIIPRTEVEKAIRMSRSRINDAFRLNDN
SLEFLGIQPTLGPPNQPPVSIWLIVFGVVMGVIVVGIVILIFTGIRDRKKKNKARSGENPYASIDISKGENNPGFQNTDD
VQTSF

**SEQ ID NO:3**

ATGAGATTTGATGAGGACACAGGTCCAGATGATATCAGTAGTCCTCCAAATGATCACAGT
ATCCTTGTTGCTGTAACTGCTGCTCAGTCCACCATTGAGGAACAGGCCAAGACATTTTTG
GACAAGTTTAACCACGAAGCCGAAGACCTGTTCTATCAAAGTTCACTTGCTTCTTGGAAT
TATAACACCAATATTACTGAAGAGAATGTCCAAAACATGAATAATGCTGGGGACAAATGG
TCTGCCTTTTTAAAGGAACAGTCCACACTTGCCCAAATGTATCCACTACAAGAAATTCAG
AATCTCACAGTCAAGCTTCAGCTGCAGGCTCTTCAGCAAAATGGGTCTTCAGTGCTCTCA
GAAGACAAGAGCAAACGGTTGAACACAATTCTAAATACAATGAGCACCATCTACAGTACT
GGAAAAGTTTGTAACCCAGATAATCCACAAGAATGCTTATTACTTGAACCAGGTTTGAAT
GAAATAATGGCAAACAGTTTAGACTACAATGAGAGGCTCTGGGCTTGGGAAAGCTGGAGA
TCTGAGGTCGGCAAGCAGCTGAGGCCATTATATGAAGAGTATGTGGTCTTGAAAAATGAG
ATGGCAAGAGCAAATCATTATGAGGACTATGGGGATTATTGGAGAGGAGACTATGAAGTA
AATGGGGTAGATGGCTATGACTACAGCCGCGGCCAGTTGATTGAAGATGTGGAACATACC
TTTGAAGAGATTAAACCATTATATGAACATCTTCATGCCTATGTGAGGGCAAAGTTGATG
AATGCCTATCCTTCCTATATCAGTCCAATTGGATGCCTCCCTGCTCATTTGCTTGGTGAT
ATGTGGGGTAGATTTTGGACAAATCTGTACTCTTTGACAGTTCCCTTTGGACAGAAACCA
AACATAGATGTTACTGATGCAATGGTGGACCAGGCCTGGGATGCACAGAGAATATTCAAG
GAGGCCGAGAAGTTCTTTGTATCTGTTGGTCTTCCTAATATGACTCAAGGATTCTGGGAA
AATTCCATGCTAACGGACCCAGGAAATGTTCAGAAAGCAGTCTGCCATCCCACAGCTTGG
GACCTGGGGAAGGGCGACTTCAGGATCCTTATGTGCACAAAGGTGACAATGGACGACTTC
CTGACAGCTCATCATGAGATGGGGCATATCCAGTATGATATGGCATATGCTGCACAACCT
TTTCTGCTAAGAAATGGAGCTAATGAAGGATTCCATGAAGCTGTTGGGGAAATCATGTCA
CTTTCTGCAGCCACACCTAAGCATTTAAAATCCATTGGTCTTCTGTCACCCGATTTTCAA
GAAGACAATGAAACAGAAATAAACTTCCTGCTCAAACAAGCACTCACGATTGTTGGGACT
CTGCCATTTACTTACATGTTAGAGAAGTGGAGGTGGATGGTCTTTAAAGGGGAAATTCCC
AAAGACCAGTGGATGAAAAAGTGGTGGGAGATGAAATATTACACAAGGACCCTTTACCAA
TTCCAGTTTCAAGAAGCACTTTGTCAAGCAGCTAAACATGAAGGCCCTCTGCACAAATGT
GACATCTCAAACTCTACAGAAGCTGGACAGAAACTGTTCAATATGCTGAGGCTTGGAAAA
TCAGAACCCTGGACCCTAGCATTGGAAAATGTTGTAGGAGCAAAGAACATGAATGTAAGG
CCACTGCTCAACTACTTTGAGCCCTTATTTACCTGGCTGAAAGACCAGAACAAGAATTCT
TTTGTGGGATGGAGTACCGACTGGAGTCCATGGGAAGTCTTCATTCTCTTGATTGTGTCC
TCTGTGCCACAAGTGAAGATGTTTGTTTTGTTTCTCTACAGGGAGGAGGATGTGCGAGTG
GCTAATTTGAAACCAAGAATCTCCTTTAATTTCTTTGTCACTGCACCTAAAAATGTGTCT
GATATCATTCCTAGAACTGAAGTTGAAAAGGCCATCAGGATGTCCCGGAGCCGTATCAAT
GATGCTTTCCGTCTGAATGACAACAGCCTAGAGTTTCTGGGGATACAGCCAACACTTGGA
CCTCCTAACCAGCCCCCTGTTTCCATATGGCTGATTGTTTTTGGAGTTGTGATGGGAGTG
ATAGTGGTTGGCATTGTCATCCTGATCTTCACTGGGATCAGAGATCGGAAGAAGAAAAAT
AAAGCAAGAAGTGGAGAAAATCCTTATGCCTCCATCGATATTAGCAAAGGAGAAAATAAT
CCAGGATTCCAAAACACTGATGATGTTCAGACCTCCTTTTAG

**SEQ ID NO:4**

```
Query:    37 SSPPNDHSILVAVTAAQS----TIEEQAKTFLDKFNHEAEDLFYQSSLASWNYNTNITEE 204
             SS    H     T+AQS    T E +A  F+++++  ++ ++ + + A+WNYNTNIT E
Sbjct:    48 SSQTTTHQATAHQTSAQSPNLVTDEAEASKFVEEYDRTSQVVWNEYAEANWNYNTNITTE 107

Query:   205 NVQNMNNAGDKWSAFLKEQSTLAQMYPLQEIQNLTVKLQLQALQQNGSSVLSEDKSKRLN 384
               + +     + +    +  T A+ + + ++QN T+K  ++ +Q    + L   + +  N
Sbjct:   108 TSKILLQKNMQIANHTLKYGTQARKFDVNQLQNTTIKRIIKKVQDLERAALPAQELEEYN 167

Query:   385 TILNTMSTIYSTGKVCNPDNPQECLLLEPGLNEIMANSLDYNERLWAWESWRSEVGKQLR 564
              IL  M T YS   VC+P+    CL LEP L  +MA S  Y + LWAWE WR + G+ +
```

```
Sbjct:   168 KILLDMETTYSVATVCHPNG--SCLQLEPDLTNVMATSRKYEDLLWAWEGWRDKAGRAIL 225

Query:   565 PLYEEYVVLKNEMARANHYEDYGDYWRGDYEVNGVDGYDYSRGQLIEDVEHTFEEIKPLY 744
               Y +YV L N+ AR N Y D GD WR  YE   ++          +D+E  F+E++PLY
Sbjct:   226 QFYPKYVELINQAARLNGYVDAGDSWRSMYETPSLE----------QDLERLFQELQPLY 275

Query:   745 EHLHAYVRAKLMNAYPS-YISPIGCLPAHLLGDMWGRFWTNLYSLTVPFGQKPNIDVTDA 921
              +LHAYVR  L   Y + +I+  G +PAHLLG+MW + W+N+Y L VPF   P++D T+A
Sbjct:   276 LNLHAYVRRALHRHYGAQHINLEGPIPAHLLGNMWAQTWSNIYDLVVPFPSAPSMDTTEA 335

Query:   922 MVDQAWDAQRIFKEAEKFFVSVGLPNMTQGFWENSMLTDPGNVQKAVCHPTAWDLGKG-D 1098
             M+ Q W  +R+FKEA+ FF S+GL  +   FW  SML  P + ++ VCH +AWD   G D
Sbjct:   336 MLKQGWTPRRMFKEADDFFTSLGLLPVPPEFWNKSMLEKPTDGREVVCHASAWDFYNGKD 395

Query:  1099 FRILMCTKVTMDDFLTAHHEMGHIQYDMAYAAQPFLLRNGANEGFHEAVGEIMSLSAATP 1278
             FRI  CT V ++D + AHHEMGHIQY M Y   P  LR GAN GFHEA+G++++LS +TP
Sbjct:   396 FRIKQCTTVNLEDLVVAHHEMGHIQYFMQYKDLPVALREGANPGFHEAIGDVLALSVSTP 455

Query:  1279 KHLKSIGLLSPDFQEDNETEINFLLKQALTIVGTLPFTYMLEKWRWMVFKGEIPKDQWMK 1458
             KHL S+ LLS +   D E +INFL+K AL  +  +PF+Y++++WRW VF G I K+ + +
Sbjct:   456 KHLHSLNLLSSEGGSD-EHDINFLMKMALDKIAFIPFSYLVDQWRWRVFDGSITKENYNQ 514

Query:  1459 KWWEMK 1476
             +WW ++
Sbjct:   515 EWWSLR 520
```

SEQ ID NO:4 refers to the amino acid sequence labeled "Query"

## Claims

1. An isolated polypeptide comprising an amino acid sequence which has at least 70% identity to the amino acid sequence of SEQ ID NO:2 over the entire length of of SEQ ID NO:2.

2. An isolated polypeptide as claimed in claim 1 in which the amino acid sequence has at least 95% identity.

3. The polypeptide as claimed in claim 1 comprising the amino acid sequence of SEQ ID NO:2.

4. The isolated polypeptide of SEQ ID NO:2.

5. An isolated polynucleotide comprising a nucleotide sequence encoding a polypeptide that has at least 70% identity to the amino acid sequence of SEQ ID NO:2, over the entire length of SEQ ID NO:2; or a nucleotide sequence complementary to said isolated polynucleotide.

**6.** An isolated polynucleotide which comprises a nucleotide sequence which has at least 70% identity to that of SEQ ID NO: 1 over the entire length of SEQ ID NO:1; or a nucleotide sequence complementary to said isolated polynucleotide.

**7.** The isolated polynucleotide as claimed in any one of claims 5 or 6 in which the identity is at least 95%.

**8.** An isolated polynucleotide selected from:

(a) a polynucleotide comprising a nucleotide sequence encoding the polypeptide of SEQ ID NO:2; and
(b) the polynucleotide of SEQ ID NO:1,

or a nucleotide sequence complementary to said isolated polynucleotide

**9.** An expression system comprising a polynucleotide capable of producing a polypeptide of claim 1 when said expression system is present in a compatible host cell.

**10.** A host cell comprising the expression system of claim 9 or a membrane thereof expressing the polypeptide of claim 1.

**11.** A process for producing a polypeptide of claim 1 comprising culturing a host cell of claim 10 under conditions sufficient for the production of said polypeptide and recovering the polypeptide from the culture medium.

**12.** An antibody immunospecific for the polypeptide of claim 1.

**13.** A method for screening to identify compounds which stimulate or which inhibit the function of the polypeptide of claim 1 which comprises a method selected from the group consisting of:

(a) measuring the binding of a candidate compound to the polypeptide (or to the cells or membranes bearing the polypeptide) or a fusion protein thereof by means of a label directly or indirectly associated with the candidate compound;
(b) measuring the binding of a candidate compound to the polypeptide (or to the cells or membranes bearing the polypeptide) or a fusion protein thereof in the presense of a labeled competitior;
(c) testing whether the candidate compound results in a signal generated by activation or inhibition of the polypeptide, using detection systems appropriate to the cells or cell membranes bearing the polypeptide;
(d) mixing a candidate compound with a solution containing a polypeptide of claim 1, to form a mixture, measuring activity of the polypeptide in the mixture, and comparing the activity of the mixture to a standard; or
(e) detecting the effect of a candidate compound on the production of mRNA encoding said polypeptide and said polypeptide in cells, using for instance, an ELISA assay.

**14.** An isolated polynucleotide selected form the group consisting of:

(a) an isolated polynucleotide comprising a nucleotide sequence which has at least 70% identity to SEQ ID NO:3 over the entire length of SEQ ID NO:3;
(b) an isolated polynucleotide comprising the polynucleotide of SEQ ID NO:3;
(c) the polynucleotide of SEQ ID NO:3; or
(d) an isolated polynucleotide comprising a nucleotide sequence encoding a polypeptide which has at least 70% identity to the amino acid sequence of SEQ ID NO:4, over the entire length of SEQ ID NO:4.

**15.** A polypeptide selected from the group consisting of:

(a) a polypeptide which comprises an amino acid sequence which has at least 70% identity to that of SEQ ID NO:4 over the entire length of SEQ ID NO:4;
(b) a polypeptide in which the amino acid sequence has at least 70% identity to the amino acid sequence of SEQ ID NO:4 over the entire length of SEQ ID NO:4;
(c) a polypeptide which comprises the amino acid of SEQ ID NO:4;
(d) a polypeptide which is the polypeptide of SEQ ID NO:4; or

(e) a polypeptide which is encoded by a polynucleotide comprising the sequence contained in SEQ ID NO:3.

**Revendications**

1. Polypeptide isolé comprenant une séquence d'aminoacides qui présente une identité d'au moins 70 % avec la séquence d'aminoacides de la SEQ ID N°2 sur la totalité de la longueur de la SEQ ID N°2.

2. Polypeptide isolé suivant la revendication 1, dans lequel la séquence d'aminoacides présente une identité d'au moins 95 %.

3. Polypeptide suivant la revendication 1, comprenant la séquence d'aminoacides de la SEQ ID N°2.

4. Polypeptide isolé de la SEQ ID N°2.

5. Polynucléotide isolé comprenant une séquence de nucléotides codant pour un polypeptide qui présente une identité d'au moins 70 % avec la séquence d'aminoacides de la SEQ ID N°2, sur la totalité de la longueur de la SEQ ID N°2 ; ou une séquence de nucléotides complémentaire dudit polynucléotide isolé.

6. Polynucléotide isolé qui comprend une séquence de nucléotides qui présente une identité d'au moins 70 % avec celui de la SEQ ID N°1 sur la totalité de la longueur de la SEQ ID N°1 ; ou une séquence de nucléotides complémentaire dudit polynucléotide isolé.

7. Polynucléotide isolé suivant l'une quelconque des revendications 5 et 6, dans lequel l'identité est d'au moins 95 %.

8. Polynucléotide isolé choisi entre :

   (a) un polynucléotide comprenant une séquence de nucléotides codant pour le polypeptide de la SEQ ID N°2 ; et
   (b) le polynucléotide de la SEQ ID N°1,

   ou une séquence de nucléotides complémentaire dudit polynucléotide isolé.

9. Système d'expression comprenant un polynucléotide capable de produire un polypeptide de la revendication 1 lorsque ledit système d'expression est présent dans une cellule hôte compatible.

10. Cellule hôte comprenant le système d'expression de la revendication 9, ou membrane de celle-ci, exprimant le polypeptide de la revendication 1.

11. Procédé pour la production d'un polypeptide de la revendication 1, comprenant les étapes consistant à cultiver une cellule hôte de la revendication 10 dans des conditions suffisantes pour la production dudit polypeptide et à recueillir le polypeptide à partir du milieu de culture.

12. Anticorps immunospécifique contre le polypeptide de la revendication 1.

13. Procédé de sélection pour identifier des composés qui stimulent ou qui inhibent la fonction du polypeptide de la revendication 1, qui comprend un procédé choisi dans le groupe consistant en :

   (a) la mesure de la liaison d'un composé candidat au polypeptide (ou aux cellules ou membranes portant le polypeptide) ou d'une protéine de fusion de celui-ci au moyen d'un marqueur associé directement ou indirectement au composé candidat ;
   (b) la mesure de la liaison d'un composé candidat au polypeptide (ou aux cellules ou membranes portant le polypeptide) ou d'une protéine de fusion de celui-ci en présence d'un compétiteur marqué ;
   (c) un test pour déterminer si le composé candidat a pour résultat un signal engendré par activation ou inhibition du polypeptide, en utilisant des systèmes de détection appropriés aux cellules ou membranes cellulaires portant le polypeptide ;
   (d) le mélange d'un composé candidat à une solution contenant un polypeptide de la revendication 1, pour former un mélange, la mesure de l'activité du polypeptide dans le mélange et la comparaison de l'activité du mélange à celle d'un échantillon de référence ; ou
(e) la détection de l'effet d'un composé candidat sur la production de l'ARNm codant pour ledit polypeptide et dudit polypeptide dans des cellules, en utilisant, par exemple, une analyse ELISA.

**14.** Polynucléotide isolé choisi dans le groupe consistant en :

(a) un polynucléotide isolé comprenant une séquence de nucléotides qui présente une identité d'au moins 70 % avec la SEQ ID N°3 sur la totalité de la longueur de la SEQ ID N°3 ;
(b) un polynucléotide isolé comprenant le polynucléotide de la SEQ ID N°3 ;
(c) le polynucléotide de la SEQ ID N°3 ; ou
(d) un polynucléotide isolé comprenant une séquence de nucléotides codant pour un polypeptide qui présente une identité d'au moins 70 % avec la séquence d'aminoacides de la SEQ ID N°4, sur la totalité de la longueur de la SEQ ID N°4.

**15.** Polypeptide choisi dans le groupe consistant en :

(a) un polypeptide qui comprend une séquence d'aminoacides qui présente une identité d'au moins 70 % avec celui de la SEQ ID N°4 sur la totalité de la longueur de la SEQ ID N°4 ;
(b) un polypeptide dans lequel la séquence d'aminoacides présente une identité d'au moins 70 % avec la séquence d'aminoacides de la SEQ ID N°4 sur la totalité de la longueur de la SEQ ID N°4 ;
(c) un polypeptide qui comprend l'aminoacide de la SEQ ID N°4 ;
(d) un polypeptide qui est le polypeptide de la SEQ ID N°4 ; ou
(e) un polypeptide qui est codé par un polynucléotide comprenant la séquence présente dans la SEQ ID N°3.

## Patentansprüche

**1.** Isoliertes Polypeptid, umfassend eine Aminosäuresequenz, die mit der Aminosäuresequenz von SEQ ID NO: 2 eine Identität von mindestens 70% über die gesamte Länge von SEQ ID NO: 2 aufweist.

**2.** Isoliertes Polypeptid gemäß Anspruch 1, wobei die Aminosäuresequenz eine Identität von mindestens 95% aufweist.

**3.** Polypeptid gemäß Anspruch 1, umfassend die Aminosäuresequenz von SEQ ID NO: 2.

**4.** Isoliertes Polypeptid gemäß SEQ ID NO: 2.

**5.** Isoliertes Polynucleotid, umfassend eine Nucleotidsequenz, die ein Polypeptid codiert, das mit der Aminosäuresequenz von SEQ ID NO: 2 eine Identität von mindestens 70% über die gesamte Länge von SEQ ID NO: 2 aufweist; oder eine Nucleotidsequenz, die zu dem isolierten Polynucleotid komplementär ist.

**6.** Isoliertes Polynucleotid, das eine Nucleotidsequenz umfasst, die mit der Nucleotidsequenz von SEQ ID NO: 1 eine Identität von mindestens 70% über die gesamte Länge von SEQ ID NO: 1 aufweist, oder eine Nucleotidsequenz, die zu dem isolierten Polynucleotid komplementär ist.

**7.** Isoliertes Polynucleotid gemäß einem der Ansprüche 5 oder 6, wobei die Identität mindestens 95% ist.

**8.** Isoliertes Polynucleotid ausgewählt aus:

(a) einem Polynucleotid, umfassend eine Nucleotidsequenz, die das Polypeptid von SEQ ID NO: 2 codiert; und
(b) dem Polynucleotid von SEQ ID NO: 1,

oder eine Nucleotidsequenz, die zu dem isolierten Polynucleotid komplementär ist.

**9.** Expressionssystem, umfassend ein Polynucleotid, das in der Lage ist, ein Polypeptid gemäß Anspruch 1 zu produzieren, wenn das Expressionssystem in einer kompatiblen Wirtszelle vorliegt.

**10.** Wirtszelle, umfassend das Expressionssystem gemäß Anspruch 9 oder eine Membran hiervon, das/die das Polypeptid gemäß Anspruch 1 exprimiert.

**11.** Verfahren zur Herstellung eines Polypeptids gemäß Anspruch 1, umfassend das Züchten einer Wirtszelle gemäß Anspruch 10 unter Bedingungen, die ausreichend sind für die Herstellung des Polypeptids, und das Gewinnen des Polypeptids aus dem Kulturmedium.

**12.** Antikörper, der für das Polypeptid gemäß Anspruch 1 immunspezifisch ist.

**13.** Verfahren zur Durchmusterung, um Verbindungen zu identifizieren, die die Funktion des Polypeptids gemäß Anspruch 1 stimulieren oder inhibieren, das ein Verfahren umfasst, das ausgewählt ist aus der Gruppe bestehend aus:

(a) Messen der Bindung einer Kandidatenverbindung an das Polypeptid (oder an die Zellen oder Membranen, die das Polypeptid tragen) oder an ein Fusionsprotein hiervon unter Verwendung einer Markierung, die mit der Kandidatenverbindung direkt oder indirekt assoziiert ist;
(b) Messen der Bindung einer Kandidatenverbindung an das Polypeptid (oder an die Zellen oder Membranen, die das Polypeptid tragen) oder an ein Fusionsprotein hiervon in Anwesenheit eines markierten Kompetitors;
(c) Nachweisen, ob die Kandidatenverbindung ein Signal auslöst, das durch Aktivierung oder Inhibierung des Polypeptids erzeugt wird, unter Verwendung von Detektionssystemen, die für die Zellen oder Zellmembranen, die das Polypeptid tragen, geeignet sind;
(d) Mischen einer Kandidatenverbindung mit einer Lösung, die ein Polypeptid gemäß Anspruch 1 enthält, um eine Mischung zu erhalten, Messen der Aktivität des Polypeptids in der Mischung und Vergleichen der Aktivität der Mischung mit einem Standard; oder
(e) Nachweisen der Wirkung einer Kandidatenverbindung auf die Produktion von mRNA, die das Polypeptid codiert, und des Polypeptids in Zellen, beispielsweise unter Verwendung eines ELISA-Tests.

**14.** Isoliertes Polynucleotid, ausgewählt aus der Gruppe bestehend aus:

(a) einem isolierten Polynucleotid, umfassend eine Nucleotidsequenz, die mit SEQ ID NO: 3 eine Identität von mindestens 70% über die gesamte Länge von SEQ ID NO: 3 aufweist;
(b) einem isolierten Polynucleotid, umfassend das Polynucleotid gemäß SEQ ID NO: 3;
(c) dem Polynucleotid von SEQ ID NO: 3; oder
(d) einem isolierten Polynucleotid, umfassend eine Nucleotidsequenz, die ein Polypeptid codiert, das mit der Aminosäuresequenz von SEQ ID NO: 4 eine Identität von mindestens 70% über die gesamte Länge von SEQ ID NO: 4 aufweist.

**15.** Polypeptid, ausgewählt aus der Gruppe bestehend aus:

(a) einem Polypeptid, das eine Aminosäuresequenz umfasst, die mit der Aminosäuresequenz von SEQ ID NO: 4 eine Identität von mindestens 70% über die gesamte Länge von SEQ ID NO: 4 aufweist;
(b) einem Polypeptid, dessen Aminosäuresequenz mit der Aminosäuresequenz von SEQ ID NO: 4 eine Identität von mindestens 70% über die gesamte Länge von SEQ ID NO: 4 aufweist;
(c) einem Polypeptid, das die Aminosäuresequenz von SEQ ID NO: 4 umfasst;
(d) einem Polypeptid, das das Polypeptid von SEQ ID NO: 4 ist; oder
(e) einem Polypeptid, das von einem Polynucleotid codiert wird, das die in SEQ ID NO: 3 enthaltene Sequenz umfasst.

SEQUENCE LISTING

<110> SmithKline Beecham plc

<120> Novel Compounds

<130> GP30129

<160> 4

<170> FastSEQ for Windows Version 3.0

<210> 1
<211> 2418
<212> DNA
<213> Homo sapiens

<400> 1

```
atgtcaagct cttcctggct ccttctcagc cttgttgctg taactgctgc tcagtccacc      60
attgaggaac aggccaagac atttttggac aagtttaacc acgaagccga agacctgttc     120
tatcaaagtt cacttgcttc ttggaattat aacaccaata ttactgaaga gaatgtccaa     180
aacatgaata atgctgggga caaatggtct gcctttttaa aggaacagtc cacacttgcc     240
caaatgtatc cactacaaga aattcagaat ctcacagtca agcttcagct gcaggctctt     300
cagcaaaatg ggtcttcagt gctctcagaa gacaagagca aacggttgaa cacaattcta     360
aatacaatga gcaccatcta cagtactgga aaagtttgta acccagataa tccacaagaa     420
tgcttattac ttgaaccagg tttgaatgaa ataatggcaa acagtttaga ctacaatgag     480
aggctctggg cttgggaaag ctggagatct gaggtcggca agcagctgag gccattatat     540
gaagagtatg tggtcttgaa aaatgagatg gcaagagcaa atcattatga ggactatggg     600
gattattgga gaggagacta tgaagtaaat ggggtagatg gctatgacta cagccgcggc     660
cagttgattg aagatgtgga acatacettt gaagagatta aaccattata tgaacatctt     720
catgcctatg tgagggcaaa gttgatgaat gcctatcctt cctatatcag tccaattgga     780
tgcctccctg ctcatttgct tggtgatatg tggggtagat tttggacaaa tctgtactct     840
ttgacagttc cctttggaca gaaaccaaac atagatgtta ctgatgcaat ggtggaccag     900
gcctgggatg cacagagaat attcaaggag gccgagaagt tctttgtatc tgttggtctt     960
cctaatatga ctcaaggatt ctgggaaaat tccatgctaa cggacccagg aaatgttcag    1020
aaagcagtct gccatcccac agcttgggac ctggggaagg gcgacttcag gatccttatg    1080
tgcacaaagg tgacaatgga cgacttcctg acagctcatc atgagatggg gcatatccag    1140
tatgatatgg catatgctgc acaacctttt ctgctaagaa atggagctaa tgaaggattc    1200
catgaagctg ttggggaaat catgtcactt tctgcagcca cacctaagca tttaaaatcc    1260
attggtcttc tgtcacccga ttttcaagaa gacaatgaaa cagaaataaa cttcctgctc    1320
```

```
aaacaagcac tcacgattgt tgggactctg ccatttactt acatgttaga gaagtggagg   1380
tggatggtct ttaaagggga aattcccaaa gaccagtgga tgaaaaagtg gtgggagatg   1440
aagcgagaga tagttggggt ggtggaacct gtgccccatg atgaaacata ctgtgacccc   1500
gcatctctgt tccatgtttc taatgattac tcattcattc gatattacac aaggaccctt   1560
taccaattcc agtttcaaga agcactttgt caagcagcta aacatgaagg ccctctgcac   1620
aaatgtgaca tctcaaactc tacagaagct ggacagaaac tgttcaatat gctgaggctt   1680
ggaaaatcag aaccctggac cctagcattg gaaaatgttg taggagcaaa gaacatgaat   1740
gtaaggccac tgctcaacta ctttgagccc ttatttacct ggctgaaaga ccagaacaag   1800
aattcttttg tgggatggag taccgactgg agtccatatg cagaccaaag catcaaagtg   1860
aggataagcc taaaatcagc tcttggagat aaagcatatg aatggaacga caatgaaatg   1920
tacctgttcc gatcatctgt tgcatatgct atgaggcagt actttttaaa agtaaaaaat   1980
cagatgattc tttttgggga ggaggatgtg cgagtggcta atttgaaacc aagaatctcc   2040
tttaatttct ttgtcactgc acctaaaaat gtgtctgata tcattcctag aactgaagtt   2100
gaaaaggcca tcaggatgtc ccggagccgt atcaatgatg ctttccgtct gaatgacaac   2160
agcctagagt ttctggggat acagccaaca cttggacctc ctaaccagcc ccctgtttcc   2220
atatggctga ttgtttttgg agttgtgatg ggagtgatag tggttggcat tgtcatcctg   2280
atcttcactg ggatcagaga tcggaagaag aaaaataaag caagaagtgg agaaaatcct   2340
tatgcctcca tcgatattag caaaggagaa aataatccag gattccaaaa cactgatgat   2400
gttcagacct ccttttag                                                 2418
```

<210> 2
<211> 805
<212> PRT
<213> Homo sapiens

<400> 2

```
Met Ser Ser Ser Ser Trp Leu Leu Leu Ser Leu Val Ala Val Thr Ala
1               5                   10                  15
Ala Gln Ser Thr Ile Glu Glu Gln Ala Lys Thr Phe Leu Asp Lys Phe
            20                  25                  30
Asn His Glu Ala Glu Asp Leu Phe Tyr Gln Ser Ser Leu Ala Ser Trp
        35                  40                  45
Asn Tyr Asn Thr Asn Ile Thr Glu Glu Asn Val Gln Asn Met Asn Asn
    50                  55                  60
Ala Gly Asp Lys Trp Ser Ala Phe Leu Lys Glu Gln Ser Thr Leu Ala
65                  70                  75                  80
Gln Met Tyr Pro Leu Gln Glu Ile Gln Asn Leu Thr Val Lys Leu Gln
                85                  90                  95
Leu Gln Ala Leu Gln Gln Asn Gly Ser Ser Val Leu Ser Glu Asp Lys
            100                 105                 110
Ser Lys Arg Leu Asn Thr Ile Leu Asn Thr Met Ser Thr Ile Tyr Ser
        115                 120                 125
```

Thr Gly Lys Val Cys Asn Pro Asp Asn Pro Gln Glu Cys Leu Leu Leu
130 135 140

Glu Pro Gly Leu Asn Glu Ile Met Ala Asn Ser Leu Asp Tyr Asn Glu
145 150 155 160

Arg Leu Trp Ala Trp Glu Ser Trp Arg Ser Glu Val Gly Lys Gln Leu
165 170 175

Arg Pro Leu Tyr Glu Glu Tyr Val Val Leu Lys Asn Glu Met Ala Arg
180 185 190

Ala Asn His Tyr Glu Asp Tyr Gly Asp Tyr Trp Arg Gly Asp Tyr Glu
195 200 205

Val Asn Gly Val Asp Gly Tyr Asp Tyr Ser Arg Gly Gln Leu Ile Glu
210 215 220

Asp Val Glu His Thr Phe Glu Glu Ile Lys Pro Leu Tyr Glu His Leu
225 230 235 240

His Ala Tyr Val Arg Ala Lys Leu Met Asn Ala Tyr Pro Ser Tyr Ile
245 250 255

Ser Pro Ile Gly Cys Leu Pro Ala His Leu Leu Gly Asp Met Trp Gly
260 265 270

Arg Phe Trp Thr Asn Leu Tyr Ser Leu Thr Val Pro Phe Gly Gln Lys
275 280 285

Pro Asn Ile Asp Val Thr Asp Ala Met Val Asp Gln Ala Trp Asp Ala
290 295 300

Gln Arg Ile Phe Lys Glu Ala Glu Lys Phe Phe Val Ser Val Gly Leu
305 310 315 320

Pro Asn Met Thr Gln Gly Phe Trp Glu Asn Ser Met Leu Thr Asp Pro
325 330 335

Gly Asn Val Gln Lys Ala Val Cys His Pro Thr Ala Trp Asp Leu Gly
340 345 350

Lys Gly Asp Phe Arg Ile Leu Met Cys Thr Lys Val Thr Met Asp Asp
355 360 365

Phe Leu Thr Ala His His Glu Met Gly His Ile Gln Tyr Asp Met Ala
370 375 380

Tyr Ala Ala Gln Pro Phe Leu Leu Arg Asn Gly Ala Asn Glu Gly Phe
385 390 395 400

His Glu Ala Val Gly Glu Ile Met Ser Leu Ser Ala Ala Thr Pro Lys
405 410 415

His Leu Lys Ser Ile Gly Leu Leu Ser Pro Asp Phe Gln Glu Asp Asn
420 425 430

Glu Thr Glu Ile Asn Phe Leu Leu Lys Gln Ala Leu Thr Ile Val Gly
435 440 445

Thr Leu Pro Phe Thr Tyr Met Leu Glu Lys Trp Arg Trp Met Val Phe
450 455 460

Lys Gly Glu Ile Pro Lys Asp Gln Trp Met Lys Lys Trp Trp Glu Met
465 470 475 480
Lys Arg Glu Ile Val Gly Val Val Glu Pro Val Pro His Asp Glu Thr
485 490 495
Tyr Cys Asp Pro Ala Ser Leu Phe His Val Ser Asn Asp Tyr Ser Phe
500 505 510
Ile Arg Tyr Tyr Thr Arg Thr Leu Tyr Gln Phe Gln Phe Gln Glu Ala
515 520 525
Leu Cys Gln Ala Ala Lys His Glu Gly Pro Leu His Lys Cys Asp Ile
530 535 540
Ser Asn Ser Thr Glu Ala Gly Gln Lys Leu Phe Asn Met Leu Arg Leu
545 550 555 560
Gly Lys Ser Glu Pro Trp Thr Leu Ala Leu Glu Asn Val Val Gly Ala
565 570 575
Lys Asn Met Asn Val Arg Pro Leu Leu Asn Tyr Phe Glu Pro Leu Phe
580 585 590
Thr Trp Leu Lys Asp Gln Asn Lys Asn Ser Phe Val Gly Trp Ser Thr
595 600 605
Asp Trp Ser Pro Tyr Ala Asp Gln Ser Ile Lys Val Arg Ile Ser Leu
610 615 620
Lys Ser Ala Leu Gly Asp Lys Ala Tyr Glu Trp Asn Asp Asn Glu Met
625 630 635 640
Tyr Leu Phe Arg Ser Ser Val Ala Tyr Ala Met Arg Gln Tyr Phe Leu
645 650 655
Lys Val Lys Asn Gln Met Ile Leu Phe Gly Glu Glu Asp Val Arg Val
660 665 670
Ala Asn Leu Lys Pro Arg Ile Ser Phe Asn Phe Phe Val Thr Ala Pro
675 680 685
Lys Asn Val Ser Asp Ile Ile Pro Arg Thr Glu Val Glu Lys Ala Ile
690 695 700
Arg Met Ser Arg Ser Arg Ile Asn Asp Ala Phe Arg Leu Asn Asp Asn
705 710 715 720
Ser Leu Glu Phe Leu Gly Ile Gln Pro Thr Leu Gly Pro Pro Asn Gln
725 730 735
Pro Pro Val Ser Ile Trp Leu Ile Val Phe Gly Val Val Met Gly Val
740 745 750
Ile Val Val Gly Ile Val Ile Leu Ile Phe Thr Gly Ile Arg Asp Arg
755 760 765
Lys Lys Lys Asn Lys Ala Arg Ser Gly Glu Asn Pro Tyr Ala Ser Ile
770 775 780
Asp Ile Ser Lys Gly Glu Asn Asn Pro Gly Phe Gln Asn Thr Asp Asp
785 790 795 800

Val Gln Thr Ser Phe
805

<210> 3
<211> 2262
<212> DNA
<213> Homo sapiens

<400> 3

```
atgagatttg atgaggacac aggtccagat gatatcagta gtcctccaaa tgatcacagt      60
atccttgttg ctgtaactgc tgctcagtcc accattgagg aacaggccaa gacatttttg     120
gacaagttta accacgaagc cgaagacctg ttctatcaaa gttcacttgc ttcttggaat     180
tataacacca atattactga agagaatgtc caaaacatga ataatgctgg ggacaaatgg     240
tctgcctttt taaaggaaca gtccacactt gcccaaatgt atccactaca agaaattcag     300
aatctcacag tcaagcttca gctgcaggct cttcagcaaa atgggtcttc agtgctctca     360
gaagacaaga gcaaacggtt gaacacaatt ctaaatacaa tgagcaccat ctacagtact     420
ggaaaagttt gtaacccaga taatccacaa gaatgcttat tacttgaacc aggtttgaat     480
gaaataatgg caaacagttt agactacaat gagaggctct gggcttggga aagctggaga     540
tctgaggtcg gcaagcagct gaggccatta tatgaagagt atgtggtctt gaaaaatgag     600
atggcaagag caaatcatta tgaggactat ggggattatt ggagaggaga ctatgaagta     660
aatggggtag atggctatga ctacagccgc ggccagttga ttgaagatgt ggaacatacc     720
tttgaagaga ttaaaccatt atatgaacat cttcatgcct atgtgagggc aaagttgatg     780
aatgcctatc cttcctatat cagtccaatt ggatgcctcc ctgctcattt gcttggtgat     840
atgtggggta gattttggac aaatctgtac tctttgacag ttccctttgg acagaaacca     900
aacatagatg ttactgatgc aatggtggac caggcctggg atgcacagag aatattcaag     960
gaggccgaga agttctttgt atctgttggt cttcctaata tgactcaagg attctgggaa    1020
aattccatgc taacggaccc aggaaatgtt cagaaagcag tctgccatcc cacagcttgg    1080
gacctgggga agggcgactt caggatcctt atgtgcacaa aggtgacaat ggacgacttc    1140
ctgacagctc atcatgagat ggggcatatc cagtatgata tggcatatgc tgcacaacct    1200
tttctgctaa gaaatggagc taatgaagga ttccatgaag ctgttgggga aatcatgtca    1260
ctttctgcag ccacacctaa gcatttaaaa tccattggtc ttctgtcacc cgattttcaa    1320
gaagacaatg aaacagaaat aaacttcctg ctcaaacaag cactcacgat tgttgggact    1380
ctgccattta cttacatgtt agagaagtgg aggtggatgg tctttaaagg ggaaattccc    1440
aaagaccagt ggatgaaaaa gtggtgggag atgaaatatt acacaaggac cctttaccaa    1500
ttccagtttc aagaagcact ttgtcaagca gctaaacatg aaggccctct gcacaaatgt    1560
gacatctcaa actctacaga agctggacag aaactgttca atatgctgag gcttggaaaa    1620
tcagaaccct ggaccctagc attggaaaat gttgtaggag caaagaacat gaatgtaagg    1680
ccactgctca actactttga gcccttattt acctggctga aagaccagaa caagaattct    1740
tttgtgggat ggagtaccga ctggagtcca tgggaagtct tcattctctt gattgtgtcc    1800
tctgtgccac aagtgaagat gtttgttttg tttctctaca gggaggagga tgtgcgagtg    1860
gctaatttga aaccaagaat ctcctttaat ttctttgtca ctgcacctaa aaatgtgtct    1920
gatatcattc ctagaactga agttgaaaag gccatcagga tgtcccggag ccgtatcaat    1980
```

```
gatgctttcc gtctgaatga caacagccta gagtttctgg ggatacagcc aacacttgga     2040
cctcctaacc agccccctgt ttccatatgg ctgattgttt ttggagttgt gatgggagtg     2100
atagtggttg gcattgtcat cctgatcttc actgggatca gagatcggaa gaagaaaaat     2160
aaagcaagaa gtggagaaaa tccttatgcc tccatcgata ttagcaaagg agaaaataat     2220
ccaggattcc aaaacactga tgatgttcag acctcctttt ag                        2262
```

<210> 4
<211> 480
<212> PRT
<213> Homo sapiens

<400> 4

```
Ser Ser Pro Pro Asn Asp His Ser Ile Leu Val Ala Val Thr Ala Ala
1               5                   10                  15
Gln Ser Thr Ile Glu Glu Gln Ala Lys Thr Phe Leu Asp Lys Phe Asn
            20                  25                  30
His Glu Ala Glu Asp Leu Phe Tyr Gln Ser Ser Leu Ala Ser Trp Asn
        35                  40                  45
Tyr Asn Thr Asn Ile Thr Glu Glu Asn Val Gln Asn Met Asn Asn Ala
    50                  55                  60
Gly Asp Lys Trp Ser Ala Phe Leu Lys Glu Gln Ser Thr Leu Ala Gln
65                  70                  75                  80
Met Tyr Pro Leu Gln Glu Ile Gln Asn Leu Thr Val Lys Leu Gln Leu
                85                  90                  95
Gln Ala Leu Gln Gln Asn Gly Ser Ser Val Leu Ser Glu Asp Lys Ser
            100                 105                 110
Lys Arg Leu Asn Thr Ile Leu Asn Thr Met Ser Thr Ile Tyr Ser Thr
        115                 120                 125
Gly Lys Val Cys Asn Pro Asp Asn Pro Gln Glu Cys Leu Leu Leu Glu
    130                 135                 140
Pro Gly Leu Asn Glu Ile Met Ala Asn Ser Leu Asp Tyr Asn Glu Arg
145                 150                 155                 160
Leu Trp Ala Trp Glu Ser Trp Arg Ser Glu Val Gly Lys Gln Leu Arg
                165                 170                 175
Pro Leu Tyr Glu Glu Tyr Val Val Leu Lys Asn Glu Met Ala Arg Ala
            180                 185                 190
Asn His Tyr Glu Asp Tyr Gly Asp Tyr Trp Arg Gly Asp Tyr Glu Val
        195                 200                 205
Asn Gly Val Asp Gly Tyr Asp Tyr Ser Arg Gly Gln Leu Ile Glu Asp
    210                 215                 220
Val Glu His Thr Phe Glu Glu Ile Lys Pro Leu Tyr Glu His Leu His
225                 230                 235                 240
```

Ala Tyr Val Arg Ala Lys Leu Met Asn Ala Tyr Pro Ser Tyr Ile Ser
245 250 255

Pro Ile Gly Cys Leu Pro Ala His Leu Leu Gly Asp Met Trp Gly Arg
260 265 270

Phe Trp Thr Asn Leu Tyr Ser Leu Thr Val Pro Phe Gly Gln Lys Pro
275 280 285

Asn Ile Asp Val Thr Asp Ala Met Val Asp Gln Ala Trp Asp Ala Gln
290 295 300

Arg Ile Phe Lys Glu Ala Glu Lys Phe Phe Val Ser Val Gly Leu Pro
305 310 315 320

Asn Met Thr Gln Gly Phe Trp Glu Asn Ser Met Leu Thr Asp Pro Gly
325 330 335

Asn Val Gln Lys Ala Val Cys His Pro Thr Ala Trp Asp Leu Gly Lys
340 345 350

Gly Asp Phe Arg Ile Leu Met Cys Thr Lys Val Thr Met Asp Asp Phe
355 360 365

Leu Thr Ala His His Glu Met Gly His Ile Gln Tyr Asp Met Ala Tyr
370 375 380

Ala Ala Gln Pro Phe Leu Leu Arg Asn Gly Ala Asn Glu Gly Phe His
385 390 395 400

Glu Ala Val Gly Glu Ile Met Ser Leu Ser Ala Ala Thr Pro Lys His
405 410 415

Leu Lys Ser Ile Gly Leu Leu Ser Pro Asp Phe Gln Glu Asp Asn Glu
420 425 430

Thr Glu Ile Asn Phe Leu Leu Lys Gln Ala Leu Thr Ile Val Gly Thr
435 440 445

Leu Pro Phe Thr Tyr Met Leu Glu Lys Trp Arg Trp Met Val Phe Lys
450 455 460

Gly Glu Ile Pro Lys Asp Gln Trp Met Lys Lys Trp Trp Glu Met Lys
465 470 475 480